# EUROPEAN PATENT APPLICATION

(11) **EP 0 717 992 A2**
(43) Date of publication of application: **26.06.1996**
(21) Application number: 95309265.7
(22) Date of filing: 20.12.1995
(51) Int. Cl.: A61K 31/165, A61K 9/08

(54) **Aqueous suspension formulations for pharmaceutical applications**

(30) Priority: 21.12.1994 US 360872
(71) Applicant: McNEIL-PPC, INC., Skillman, NJ 08558 (US)
(72) Inventor: Ratnaraj, Sheila M., Telford, PA 18969 (US); Shah, Manoj N., Norristown, PA 19403 (US)
(74) Representative: Mercer, Christopher Paul

(57) **Abstract**

The invention features an aqueous pharmaceutical suspension and methods of making same. The suspension comprises a therapeutic amount of controlled release acetaminophen powder, the powder being suspended in a suspending system comprising a suspension stabilizing effective amount of xanthan gum, hydroxyethyl cellulose and pregelatinized starch, an effective amount of taste masking composition, and water.

## Description

### FIELD OF THE INVENTION

The present invention relates to aqueous pharmaceutical suspensions containing a high amount of acetaminophen embedded in a polymer to impart controlled release properties. This invention also relates to a process for preparing such suspensions.

### BACKGROUND OF THE INVENTION

Orally administered drugs are provided to the patient in many dosage forms such as tablets, capsules, solutions or suspensions. Many patients, including pediatric and geriatric patients, prefer a liquid oral dosage form to a solid dosage form. A liquid dosage is preferred by this class of patients because of the ease with which it may be swallowed. Additionally, patients may be more inclined to comply with their medication instructions if the dosages are easier to ingest.

A common problem associated with liquid pharmaceutical dosage forms is the often disagreeable taste which may manifest itself when the drug is ingested in a liquid dosage form. Acetaminophen is a drug with a bitter taste. This taste may be overcome by the addition of sweeteners or flavoring agents to the formulation which mask the bitter or unpleasant taste of the drugs. However, these agents are not completely effective in concealing the unpleasant taste of the pharmaceutical.

The duration of action of acetaminophen in the human body is approximately four hours after which the therapeutic activity of this drug declines. The therapeutic life of acetaminophen may be increased by formulating the drug in a sustained release liquid suspension. Such formulations are desirable for pediatric patients, in that parents of such children would not have to wake up in the middle of the night to dose the child. Similarly, geriatric patients would benefit from such formulations because of the ease with which they may be ingested and the elimination of the need to redose during the night. Thus, there is a need for a pharmaceutical liquid suspension of acetaminophen which has both sustained release properties and a palatable taste.

### SUMMARY OF THE INVENTION

The invention features an aqueous pharmaceutical suspension comprising a therapeutic amount of controlled release acetaminophen powder, the powder being suspended in a suspending system comprising a suspension stabilizing effective amount of xanthan gum, hydroxyethyl cellulose and pregelatinized starch, an effective amount of taste masking composition and water.

The controlled release acetaminophen powder of the invention is a Pharmasome® comprising acetaminophen and cellulose acetate butyrate or ethyl cellulose.

The invention further features a method of preparing the aqueous pharmaceutical suspension comprising mixing a therapeutic amount of controlled release acetaminophen powder in a suspending system, the suspending system comprising a suspension stabilizing effective amount of xanthan gum, hydroxyethyl cellulose and pregelatinised starch, an effective amount of taste masking composition and water.

### DETAILED DESCRIPTION

The present invention provides a stable aqueous pourable suspension for formulations containing high amounts of controlled release acetaminophen, which when combined with sweeteners and flavoring agents, provide a palatable dosage form of acetaminophen. This dosage form is also chemically stable under physiological conditions and is especially well suited for pediatric and geriatric applications.

The suspending system of the invention is suitable for suspending high concentrations of controlled release acetaminophen powder (up to 15%) in an aqueous solution. Generally the suspension may contain a total of 20 grams of acetaminophen. Preferably, acetaminophen is present in the suspension in the range of 1.0 to 15 grams per 100 ml of suspension. More preferably, the range of acetaminophen in the suspension is 3.2 to 10 grams per 100 ml of suspension.

The acetaminophen used in the present invention is in the form of a controlled release powder. Acetaminophen Pharmasomes®, which are disclosed in U.S. Patent No. 4,940, 588, and U.S. Patent No. 4,952,402, both of which are incorporated herein by reference and are commercially available from Elan Corporation, Ireland, are examples of such a powder. Acetaminophen Pharmasomes® are acetaminophen particles which are embedded in a polymer matrix of either cellulose acetate butyrate or ethyl cellulose. The term "Pharmasomes" as used herein describes microparticles containing acetaminophen embedded in a polymer matrix. By "controlled release acetaminophen powder" as used herein, is meant an acetaminophen Pharmasome® as described in U.S. Patent No. 4,940, 588, and U.S. Patent No. 4,952,402.

The polymer used in the matrix determines whether the controlled release acetaminophen powder will have an immediate or sustained release profile. In the present invention, the Pharmasome® containing the ethyl cellulose matrix exhibits an immediate release profile and the matrix is used primarily for taste-masking purposes. On the other hand, the Pharmasome® containing the cellulose acetate butyrate matrix exhibits a sustained release profile. By combining the immediate and sustained release Pharmasomes®, acetaminophen in dosed to the patient at therapeutic levels for an extended period of time, e.g., eight hours. Other release profiles can be achieved by varying the proportion of the immediate and sustained release Pharmasomes®.

The amount of the active ingredient present in the suspension should be sufficient to provide a therapeutic amount of the active ingredient in a convenient dosage unit. The acetaminophen present in the suspension is therefore in therapeutic effective amounts, which are amounts that produce the desired therapeutic response upon oral administration and can be readily determined by one skilled in the art. In determining such amounts, the particular compound being administered, the bioavailability characteristics of the pharmaceutical, the dose regimen, the age and weight of the patient and other factors must be considered.

The acetaminophen suspension of the invention employs a suspending system comprising xanthan gum, pregelatinized starch and an auxiliary suspending agent such as hydroxyethyl cellulose. Preferably, the suspending system comprises xanthan gum in the range of 0.15-0.30 gram per 100 ml of suspension, pregelatinized starch in the range of 0.65-0.80 gram per 100 ml and an auxillarly suspending agent such as hydroxyethyl cellulose comprising from about 0.45-0.60 gram per 100 ml of the suspension. More preferably, the suspending system of the invention comprises xanthan gum in the range of 0.20-0.25 gram per 100 ml of suspension, pregelatinized starch in the range of 0.70-0.77 gram per 100 ml of suspension, and hydroxyethyl cellulose in the range of 0.53-0.56 gram per 100 ml of suspension.

The xanthan gums suitable for use in the present invention are high molecular weight polysaccharides produced by *Xanthamonas campestris*. Techniques and strains for producing this polysaccharide are described in U.S. Patent No. 4,752,580 and U.S. Patent No. 3,485,719, the disclosures of which are hereby incorporated by reference. The xanthan gum used in the present invention should have a viscosity in a one percent salt solution of from about 1000 to about 1700 cP (mPA.sec.). The one percent solution's viscosity should be measured at 25°C with an LV model Brookfield Synchro-Lectric viscometer at 60 rpm, no. 3 spindle. Xanthan gum is available commercially (R.T. Vanderbilt Company and Kelco, a division of Merck). Examples of suitable xanthan gums are Keltrol™, Keltrol™ F, Keltrol™ T, Keltrol™ TF and Keltrol™ 1000 (Keltrol is a trademark of Merck, Inc.). Keltrol™, Keltrol™ F, Keltrol™ TF and Keltrol™ 1000 are the xanthan gums preferred for use in pharmaceutical suspensions.

The pregelatinized starch NF used in the suspension is prepared from modified, stabilized, waxy maize food starch. Pregelatinized starch is precooked so that it swells and begins to thicken instantly when added to cold water. Suitable commercially available pregelatinized starch is available from National Starch and Chemical Co., under the brand name ULTRA-TEX™ and ULTRA-SPERSE®.

The hydroxyethyl cellulose NF suitable for use in the present invention is a nonionic water soluble polymer derived from cellulose. By treating cellulose with sodium hydroxide and reacting with ethylene oxide, hydroxyethyl groups are introduced to yield a hydroxyethyl ether of cellulose. Suitable commercially available hydroxyethyl celluloses include NATROSOL® Brand from Aqualon Co.

The pH of the suspension of the invention is between about pH 3 and about pH 6, preferably between about pH 4.0 and about pH 5.0. Citric acid is the preferred ingredient to add to the suspension to control the pH.

As a general guideline, the particulate solids in the suspension should have a particle diameter in the range of from about 1 micron to 150 microns. Preferably, the particle diameter is in the range of from about 7 to 120 microns.

The suspension of the present invention may also include a taste masking composition to render the product more palatable. Generally the taste masking composition contains at least one sweetening agent and one flavoring agent. A coloring agent may also be added. The flavoring and coloring agents added to the mixture may be varied depending upon the preferences dictated by the intended consumer of such suspension. For example, since pediatric, adult or geriatric tastes often vary, the types of flavoring and coloring agents added to the suspension nay also vary accordingly.

Flavoring and coloring agents suitable for addition to the suspension of the invention are well known in the art. Suitable flavoring agents include natural and/or artificial flavors such as mints (i.e., peppermint, etc.,) menthol, cinnamon, vanilla, artificial vanilla, chocolate, artificial chocolate, both natural and artificial fruit flavors (i.e., cherry, grape, orange, strawberry etc.,) and combinations of two or more thereof. Flavoring agents are generally provided as a minor component of the suspension in amounts effective to provide a palatable flavor to the suspension, i.e., flavorings are generally present in the suspension in amounts in the range from about 0 grams to about 5 grams per 100 ml of the suspension.

Coloring agents are added to the suspension to provide an appealing color to the suspension. The coloring agents should be selected to avoid chemical incompatibilities with the other ingredients in the suspension. Suitable coloring agents for use in pharmaceutical suspensions are well known to those skilled in the art.

Suitable sweetening agents to be added to the suspension of the invention include, but are not limited to, sugars such as monosaccharides, disaccharides and polysaccharides. Examples of some suitable sugars include xylose, ribose, glucose, mannose, fructose, glucose, partially hydrolysed starch or corn syrup solids, and sugar alcohols such as sorbitol, xylitol, mannitol, glycerin or any combination thereof. Preferably, the sugar sweetener added to the suspension is sucrose.

The amount of sugar used in the suspension varies depending on the degree of sweetening which is desired. This may also vary depending upon the intended consumer in that pediatric formulations usually contain more sweetener than adult formulations. Generally, the amount of sugar sweetener in the suspension is in the range of about 15-90 grams per 100 ml of suspension. Preferably the amount of sugar sweetener is in the range of about 30-70 grams per 100 ml of suspension.

Water soluble artificial sweeteners may also be employed in place of or in addition to sugar sweeteners. Examples of suitable artificial sweeteners include but are not limited to aspartame, sucralose, cyclamates, saccharin and suitable combinations thereof. The amount of artificial sweetener used in the suspension may also vary depending upon the intended consumer. In general, the amount of artificial sweetener may vary from 0 to about 5 grams per 100 ml of suspension.

Wetting agents also may be added to the suspension of the invention to facilitate dispersion of hydrophobic pharmaceutically active components. The concentration of wetting agents in the suspension should be selected to achieve optimum dispersion of such pharmaceutically active components within the suspension while using the lowest feasible amount of the wetting agent. Those skilled in the art are well versed in empirical methods to determine the appropriate wetting agents and their respective concentrations which will achieve optimum dispersion of pharmaceutically active components in the suspension while avoiding flocculation within the suspension. Suitable wetting agents are listed in the USP Pharmacopeia XXI.

Preservatives are useful as additives to the suspension of the invention. Such preservatives may include sodium benzoate, potassium sorbate, salts of edetate and parabens (such as methyl, ethyl, propyl and butyl p-hydroxybenzoic acid esters). While potassium sorbate is the presently preferred preservative ingredient for addition to suspensions containing acetaminophen, other pharmaceutically acceptable preservatives may be substituted therefor.

Formulations for the sustained relief suspension of acetaminophen (8 hours) of the invention are given in Table 1.

**TABLE 1**

| **SUSTAINED RELEASE SUSPENSION OF ACETAMINOPHEN** | | |
|---|---|---|
| | BROAD RANGE (g/100 ml) | PREFERRED (g/100 ml) |
| Suspending System | | |
| Xanthan Gum | 0.15-0.30 | 0.20-0.25 |
| Pregelatinized Starch | 0.65-0.80 | 0.70-0.77 |
| Hydroxyethyl Cellulose | 0.45-0.60 | 0.53-0.56 |

| Actives | | |
|---|---|---|
| Acetaminophen | 1.0-15.0 | 3.2-10.0 |

| Other Ingredients | | |
|---|---|---|
| Sucrose, NF | 25.0-40.0 | 30.0-35.0 |
| Glycerin, USP | 1.0-10.0 | 4.0-8.0 |
| Sorbitol Solution USP (70%) | 1.0-30.0 | 10.0-25.0 |
| Polysorbate 60, NF | 0.10-0.30 | 0.20-0.25 |
| Potassium Sorbate, USP | 0.30-0.50 | 0.35-0.45 |
| Citric Acid | 0.03-0.30 | 0.10-0.25 |
| Coloring Agent | 0.001-0.05 | 0.002-0.12 |
| Flavoring Agent | 0.01-1.0 | 0.01-0.80 |
| Purified Water | 30.0-60.0 | 40.0-50.0 |

All measurements in this table are listed in grams per 100 ml of suspension as measured at 25°C. If the volume of all the components does not equal 100 ml, the additional volume may be provided by the addition of purified water.

### EXAMPLE 1. Preparation of Sustained Release Acetaminophen Suspension. An 8 hour sustained release children's acetaminophen suspension was prepared as follows:

1. To a mixing tank equipped with a High Shear mixer add the following :
   purified water USP
   sorbitol solution USP 70%.
2. Add the potassium sorbate NF and start mixing until the solid materials have dissolved.
3. In a premix tank of suitable size equipped to provide agitation, charge the glycerin USP. Reserve a portion of glycerin USP for rinsing the premix tank in step 4 below. Add xanthan gum NF and hydroxyethyl cellulose NF.
4. Immediately pump the glycerin and gums slurry prepared in Step 3 from the premix tank into the main mixing tank. Rinse the premix tank with the reserved glycerin USP.
5. Add the pregelatinized starch and continue mixing.
6. Add the sucrose NF and continue mixing.
7. Add the following:
   polysorbate 60 NF
   citric acid USP, anhydrous
   Immediate release acetaminophen Pharmasome® or Sustained release acetaminophen Pharmasome®
8. Dissolve the coloring in purified water USP and add to the mixing tank.
9. Obtain purified water USP and rinse the dye solution container. Add all rinsings and any unused water to the mixing tank.
10. Add the flavors.
11. Stop the mixer. Bring the batch to the target weight by the addition of purified water USP and complete mixing.

The composition of the resulting suspension is shown below in Table 2.

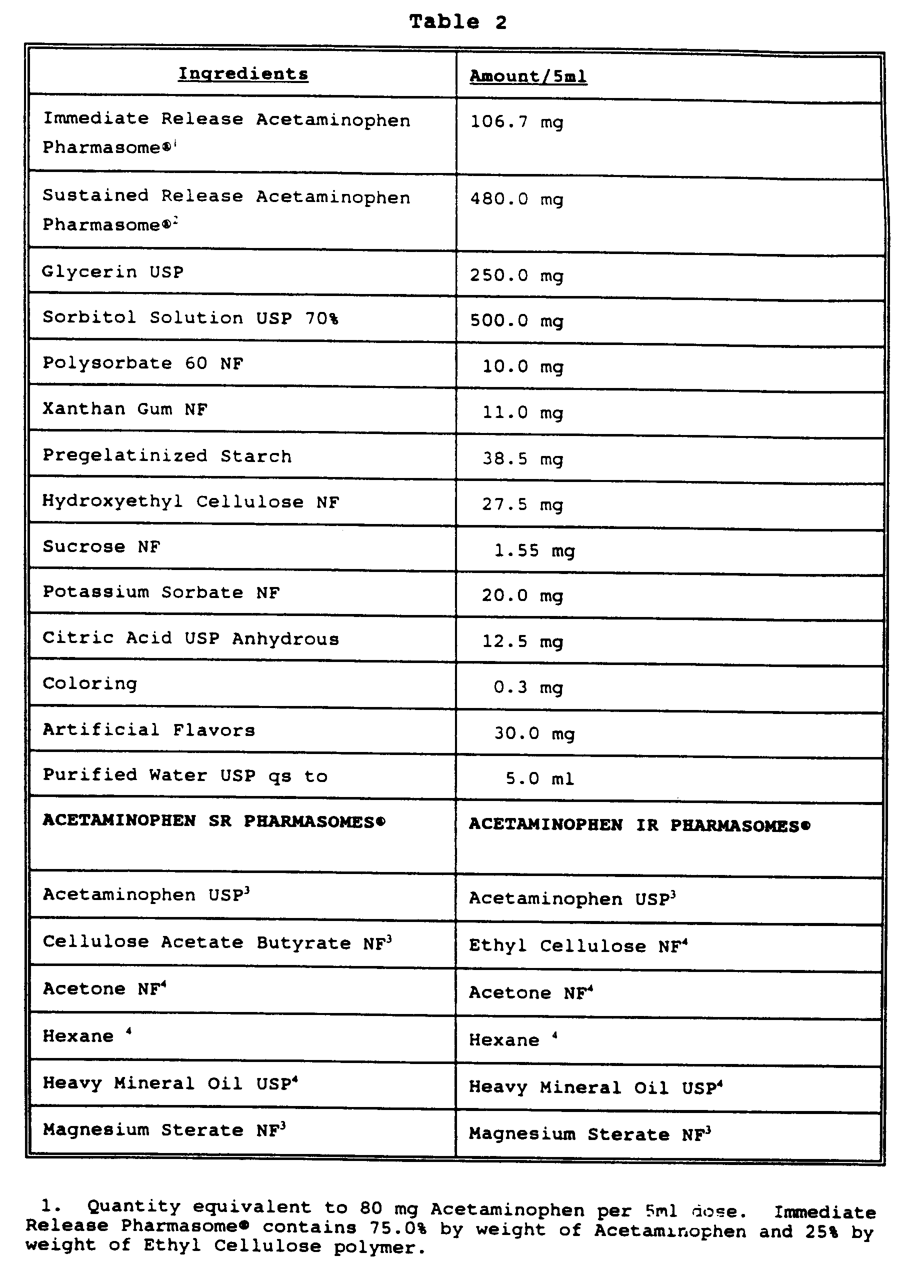

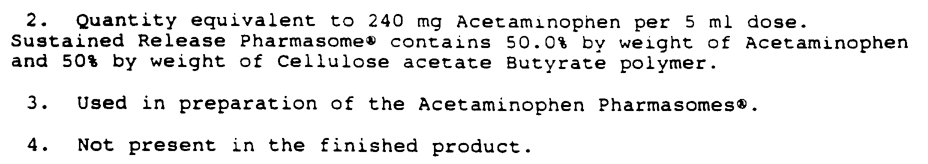

While this invention has been disclosed with reference to specific embodiments, it is apparent that other embodiments and variations of this invention may be devised by others skilled in the art without departing from the true spirit of the invention. The appended claims are intended to be construed to include all such embodiments and equivalent variations.

## Claims

1. An aqueous pharmaceutical suspension, comprising: a therapeutic amount of controlled release acetaminophen powder, said powder being suspended in a suspending system comprising a suspension stabilizing effective amount of xanthan gum, hydroxyethyl cellulose and pregelatinized starch; an effective amount of taste masking composition; and water.

2. The suspension of claim 1, wherein the concentration of said acetaminophen is up to about 20 grams per 100 ml of suspension and is preferably from 1.0 to 15.0 grams per 100 ml of suspension, most preferably from 3.2 to 10 grams per 100 ml of suspension.

3. The suspension of claim 1 or claim 2, wherein the concentration of said xanthan gum is 0.15 to 3.0 grams per 100 ml of suspension, preferably 0.20 to 0.25 grams per 100 ml of suspension.

4. The suspension of any one of claims 1 to 3, wherein the concentration of said pregelatinized starch is 0.65 to 0.80 grams per 100 ml of suspension, preferably 0.70 to 0.77 grams per 100 ml of suspension.

5. The suspension of any one of claims 1 to 4, wherein the concentration of said hydroxyethyl cellulose is 0.45 to 0.60 grams per 100 ml of suspension, preferably 0.53 to 0.56 grams per 100 ml of suspension.

6. The suspension of any one of claims 1 to 5, wherein said controlled release acetaminophen powder is a mixture of immediate and sustained release acetaminophen powders.

7. The suspension of any one of claims 1 to 6, wherein said taste masking composition comprises a sweetenerand a flavouring agent.

8. The suspension of any one of claims 1 to 7, wherein the pH of said suspension is 3 to 6, preferably 4.0 to 5.0.

9. The suspension of any one of claims 1 to 8, wherein said controlled release acetaminophen powder is a Pharmasome®, preferably comprising acetaminophen and cellulose acetate butyrate or acetaminophen and ethyl cellulose.

10. A method of preparing the aqueous pharmaceutical suspension of any one of claims 1 to 9, comprising: mixing a therapeutic amount of said controlled release acetaminophen powder with said suspending system, said taste masking composition and water.
